# EUROPEAN PATENT APPLICATION

(11) **EP 4 840 513 A2**
(43) Date of publication of application: **18.11.2026**
(21) Application number: 26209883.3
(22) Date of filing: 10.01.2023
(51) Int. Cl.: A61P 31/16

(54) **ADJUVANT COMPOSITION AND VACCINE COMPOSITION**

(30) Priority: 25.01.2022 JP 2022009225
(62) Document number(s) of the earlier application(s) in accordance with Art. 76 EPC: 23746634.7 / 4 470 561
(71) Applicant: Shin Nippon Biomedical Laboratories, Ltd., Kagoshima 891-1394 (JP)
(72) Inventor: HARUTA, Shunji, Kagoshima 891-1394 (JP)
(74) Representative: Zacco Sweden AB

(57) **Abstract**

An adjuvant composition, comprising a complex comprising an adjuvant and microparticles of a biodegradable polymer and/or cyclodextrin, wherein the adjuvant is incorporated into the microparticles of the biodegradable polymer and/or encapsulated in the cyclodextrin.

## Description

### Technical Field

The present invention relates to an adjuvant composition and a vaccine composition.

### Background Art

An adjuvant refers to a substance used to enhance the efficacy of a vaccine by being administered together with the vaccine. Recently, in order to increase the safety of vaccines, rather than administering the pathogen itself, subunit vaccines and the like which use some components of the pathogen are often used. Therefore, vaccines alone are not effective enough, and the importance of adjuvants is inevitably increasing.

Patent Literature 1 discloses, as a useful adjuvant composition, a squalene-containing oil-in-water emulsion with a particle size of less than 1 µm, obtained by emulsifying a terpenoid, oil-based squalene or the like with a surfactant such as Tween 80 or Span 85. This literature indicates that a small oil droplet form, specifically, small oil droplets where substantially all the droplets are smaller than 1 micron in diameter, preferably smaller than 0.8 micron, and more preferably smaller than 0.5 micron, is useful as an adjuvant. In this way, by making the oil-based adjuvant component into an oil-in-water emulsion, the oil-based adjuvant component and the vaccine solution do not separate when the oil-based adjuvant component is mixed with the aqueous vaccine solution, and inoculation can be performed with the oil-based adjuvant component dispersed in the aqueous vaccine solution, allowing to efficiently exhibit the effect of the adjuvant component. This squalene-containing oil-in-water emulsion is also used in a commercial influenza vaccine (trade name: FLUAD (registered trademark)) under the trade name MF-59, and has successfully reduced the amount of influenza antigen added while enhancing immunogenicity due to its adjuvant effect (Non Patent Literature 1).

This squalene-containing oil-in-water emulsion has been reported to have issues with storage stability. Specifically, it has been reported that squalene, which has many unsaturated bonds, is susceptible to degradation by oxidation, that the surfactants such as Tween 80 and Span 85 used for emulsion formation are susceptible to hydrolysis, making it difficult to maintain the emulsion form, and that the squalene content decreased when stored for 3 months at a temperature of 25 to 37°C with no pH adjuster added. Taking these stability issues into account, the FLUAD (registered trademark) influenza vaccine containing the squalene-containing oil-in-water emulsion research reagents, AddaVax^{TM} and MF-59, requires refrigerated storage and is to be discarded if frozen (Non Patent Literatures 1 and 3). On the other hand, the need to transport and store the squalene-containing oil-in-water emulsion or vaccine containing the same under refrigeration without freezing from the production facility to the location where the vaccine is administered has many issues, including increased complexity in logistics, limitations on possible vaccination sites, and complicated vaccination preparation.

The glycosphingolipid α-galactosylceramide has been reported to have adjuvant activity, but as it is water insoluble, it has been reported to be dissolved in a solution containing the surfactant Tween 20 before inoculation with an aqueous vaccine solution (Non Patent Literature 4). Such water-insoluble adjuvant components are also subjected to solubilization treatments to prevent precipitation in aqueous vaccine solutions and avoid variability in efficacy.

Thus, in order to exhibit the adjuvant effect stably and efficiently, technologies have been identified to disperse or dissolve oil-based adjuvant components or water-insoluble adjuvant components in aqueous vaccine solutions. However, as the dosage forms and formulations of vaccine preparations become increasingly diverse with the diversification of vaccine administration routes, including subcutaneous, intramuscular, intradermal, oral, and intranasal administration, there is a need for the development of adjuvant compositions that are easily applicable to new dosage forms and formulations, have storage stability, and can be dispersed or dissolved in aqueous vaccine solutions by other methods than solubilization by emulsions and surfactants. Furthermore, in order to further reduce the amount of antigen contained in vaccines and the number of vaccinations, and to improve the efficacy in newborns and the elderly who have weaker immune systems, there is a need for adjuvants with higher adjuvant activity than conventional adjuvant preparations.

### Citation List

### Patent Literature

Patent Literature 1: Japanese Patent Publication No. 8-32638 (WO 90/14837) JPH0832638(B2)

### Non Patent Literature

Non Patent Literature 1: C. B. Fox, Molecules, 2009, 14, 3286-3312.
Non Patent Literature 2: InviviGen Limited, Data Sheet for AddaVaxTM, Version 20J08-MM.
Non Patent Literature 3: Seqirus, Inc., US Package Insert for FLUAD (registered trademark), Version 14 OCT, 2020.
Non Patent Literature 4: Fotouhi, Arch Virol, 2017, 162, 1251-1260.

### Summary of Invention

### Technical Problem

An object of the present invention is to provide a novel adjuvant composition or a method for producing the same for enhancing the efficacy of a vaccine, or a vaccine composition containing the adjuvant composition.

### Solution to Problem

The present inventors have found that the efficacy of a vaccine can be improved by using a complex in which an adjuvant is incorporated into microparticles of biodegradable polymer, or by using a complex in which an adjuvant is encapsulated in cyclodextrin.

The present invention includes the following embodiments:
[1] An adjuvant composition, comprising a complex comprising an adjuvant and microparticles of a biodegradable polymer and/or cyclodextrin,
   wherein the adjuvant is incorporated into the microparticles of the biodegradable polymer and/or encapsulated in the cyclodextrin.
[2] The adjuvant composition according to [1], wherein the adjuvant comprises at least one selected from the group consisting of terpenoids and glycosphingolipids.
[3] The adjuvant composition according to [1] or [2], wherein an amount of the adjuvant is 0.1 to 40 mass% based on the complex.
[4] The adjuvant composition according to [2] or [3], wherein the terpenoid comprises at least one selected from the group consisting of retinoids and squalene.
[5] The adjuvant composition according to any of [2] to [4], wherein the glycosphingolipid comprises α-galactosylceramide.
[6] The adjuvant composition according to any of [1] to [5], wherein the biodegradable polymer comprises at least one selected from the group consisting of poly(lactic-co-glycolic acid), poly(lactic acid), and derivatives thereof.
[7] The adjuvant composition according to [6], wherein the derivative of the biodegradable polymer is a chitosan derivative, a polyethylene glycol derivative, a lysine-modified polyethylene glycol derivative, an arginine-modified polyethylene glycol derivative, or an amino group-modified polyethylene glycol derivative.
[8] The adjuvant composition according to any of [1] to [7], wherein at least one selected from the group consisting of chitosan, hexadecyltrimethylammonium, didodecyldimethylammonium, dimethyldioctadecylammonium, and salts thereof is further incorporated into the complex comprising the adjuvant and the microparticles of the biodegradable polymer.
[9] The adjuvant composition according to any of [1] to [8], wherein an average particle diameter of the complex comprising the adjuvant and the microparticles of the biodegradable polymer, is 5 to 800 nm.
[10] The adjuvant composition according to any of [1] to [9], wherein an average molecular weight of the biodegradable polymer is 5,000 to 100,000.
[11] The adjuvant composition according to any of [1] to [10], further comprising, in addition to the complex comprising the adjuvant and the microparticles of the biodegradable polymer, at least one selected from the group consisting of alum, α-cyclodextrin, β-cyclodextrin, γ-cyclodextrin, and derivatives thereof.
[12] The adjuvant composition according to any of [1] to [10], wherein the cyclodextrin comprises at least one selected from the group consisting of α-cyclodextrin, β-cyclodextrin, γ-cyclodextrin, and derivatives thereof.
[13] The adjuvant composition according to [12], wherein the derivative of the cyclodextrin is a methyl derivative, a dimethyl derivative, or a hydroxypropyl derivative.
[14] The adjuvant composition according to any of [1] to [13], further comprising at least one selected from the group consisting of crystalline cellulose, mannitol, trehalose, and sucrose.
[15] The adjuvant composition according to any of [1] to [14], which is in the form of a solid.
[16] The adjuvant composition according to any of [1] to [15], for use in injection administration or mucosal administration.
[17] A vaccine composition, comprising the adjuvant composition according to any of [1] to [16], and a vaccine.
[18] The vaccine composition according to [17], wherein the vaccine is a vaccine against influenza virus, coronavirus, RS virus, or papillomavirus.
[19] A method for producing an adjuvant composition, the method comprising incorporating an adjuvant into microparticles of a biodegradable polymer to form a complex.
[20] A method for producing an adjuvant composition, the method comprising encapsulating an adjuvant in a cyclodextrin to form a complex.

### Advantageous Effects of Invention

The present invention can provide a novel adjuvant composition or a method for producing the same for enhancing the efficacy of a vaccine, or a vaccine composition containing the adjuvant composition.

### Brief Description of Drawings

[Figure 1] Figure 1 shows the level of IFN-γ secretion (number of spots by ELISpot assay) after subcutaneous administration of OVA antigen with (or without) adjuvant composition to mice.
[Figure 2] Figure 2 shows the level of blood IgG antibody production (OD 450 absorbance) after subcutaneous administration of OVA antigen with (or without) adjuvant composition to mice.
[Figure 3] Figure 3 shows the level of blood IgG antibody production (OD 450 absorbance) after intranasal administration of OVA antigen with (or without) adjuvant composition to mice.
[Figure 4] Figure 4 shows the level of IgA antibody production (OD 450 absorbance) in bronchoalveolar lavage fluid after intranasal administration of OVA antigen with (or without) adjuvant composition to mice.
[Figure 5] Figure 5 shows the level of blood IgG antibody production (OD 450 absorbance) after subcutaneous administration of influenza HA protein antigen with (or without) adjuvant composition to mice.
[Figure 6] Figure 6 shows the level of IFN-γ secretion (number of spots by ELISpot assay) after intramuscular administration of OVA antigen with (or without) adjuvant composition to monkeys.
[Figure 7] Figure 7 shows the level of blood IgG antibody production (OD 450 absorbance) after intramuscular administration of OVA antigen with (or without) adjuvant composition to monkeys.

### Description of Embodiments

### <Adjuvant composition>

One embodiment of the present invention relates to an adjuvant composition, the composition comprising a complex comprising an adjuvant and microparticles of a biodegradable polymer and/or cyclodextrin, wherein the adjuvant is incorporated into the microparticles of the biodegradable polymer and/or encapsulated in the cyclodextrin.

When an oil-based or water-insoluble adjuvant is mixed as it is with an aqueous vaccine solution, it will separate from the aqueous vaccine solution if it is an oil-based adjuvant, or precipitate in the aqueous vaccine solution if it is a water-insoluble adjuvant. This makes it difficult to inoculate the vaccine with the adjuvant dispersed or dissolved in the aqueous vaccine solution, and may prevent the effect of the adjuvant from being efficiently exhibited.

On the other hand, in the adjuvant composition according to the present embodiment, the adjuvant is incorporated into the microparticles of a biodegradable polymer, or encapsulated in cyclodextrin, and therefore more readily disperses or dissolves in the aqueous vaccine solution, which allows to efficiently exhibit the effect of the adjuvant.

Oil-in-water emulsions containing oil-based adjuvants and surfactants require refrigerated storage while avoiding freezing in order to maintain their emulsion form.

On the other hand, the adjuvant composition according to the present embodiment can be stored in liquid form either at room temperature, refrigerated, or frozen.

In general, if the adjuvant composition can be solidified, it will have better stability than in a solution state. However, when oil-in-water emulsion adjuvant compositions such as those described in Patent Literature 1 and Non Patent Literatures 2 and 3 are solidified and then dissolved again, it is very difficult to form the original oil-in-water emulsion, which reduces the adjuvant's effect.

On the other hand, in the adjuvant composition according to the present embodiment, the complex can be readily solidified, and even if dissolved again, a complex that can disperse in an aqueous solution can be obtained. This further improves the stability of the adjuvant and allows formulation and inoculation in combination with vaccines in powder form.

The adjuvant composition according to the present embodiment exhibits a higher adjuvant effect than oil-in-water emulsion adjuvant compositions, and can therefore reduce the amount of vaccine required to enhance humoral and cellular immunity. This will allow inoculation of a larger number of people in a shorter period of time.

### [Adjuvant]

The adjuvant according to the present embodiment is preferably an oil-based or water-insoluble adjuvant.

In the present specification, "oil-based" means having the property of separating from the aqueous phase when allowed to stand after being mixed with water, and having the property of easily dissolving in oil or nonpolar solvents.

In the present specification, "water-insoluble" means having a solubility in water at 20°C of 1 mg/mL or less.

Examples of the oil-based adjuvant include a terpenoid. Examples of the terpenoid include a tocopherol, a retinoid, squalane, and squalene. Examples of the retinoid include tretinoin, retinol, retinal, allitretinoin, acitretin, etretinate, adapalene, tazarotene, and bexarotene.

Examples of the water-insoluble adjuvant include a glycosphingolipid. Examples of the glycosphingolipid include lactosylceramide, glucosylceramide, 7DW8-5, and α-galactosylceramide.

The adjuvant may be used alone or in combination of two or more.

The amount of adjuvant is preferably 0.1 to 40 mass%, more preferably 0.5 to 35 mass%, and even more preferably 5 to 30 mass% based on the complex.

### [Biodegradable polymer]

Examples of the biodegradable polymer in the present embodiment include poly(lactic-co-glycolic acid), poly(lactic acid), and derivatives thereof. Examples of the derivative include a chitosan derivative, a polyethylene glycol derivative, a lysine-modified polyethylene glycol derivative, an arginine-modified polyethylene glycol derivative, and an amino group-modified polyethylene glycol derivative.

The biodegradable polymer may be used alone or in combination of two or more.

The amount of biodegradable polymer is preferably 60 to 99.9 mass%, more preferably 65 to 99.5 mass%, and even more preferably 70 to 95 mass% based on the complex.

The average molecular weight of the biodegradable polymer is preferably 5,000 to 150,000, more preferably 5,000 to 100,000, even more preferably 5,000 to 75,000, and particularly preferably 5,000 to 50,000.

In the present specification, the "average molecular weight" is the weight-average molecular weight, and can be measured by gel permeation chromatography.

The average particle size of the complex comprising the adjuvant and the microparticles of the biodegradable polymer is preferably 5 nm to 800 nm, more preferably 10 nm to 500 nm, and even more preferably 50 nm to 200 nm. The average particle size can be measured by a dynamic light scattering method.

### [Cyclodextrin]

Examples of the cyclodextrin in the present embodiment include α-cyclodextrin, β-cyclodextrin, γ-cyclodextrin, and derivatives thereof. Examples of the derivative include a methyl derivative, a dimethyl derivative, and a hydroxypropyl derivative.

The cyclodextrin may be used alone or in combination of two or more.

The amount of cyclodextrin is preferably 60 to 99.9 mass%, more preferably 60 to 95 mass%, and even more preferably 65 to 95 mass% based on the complex.

### [Complex]

The first complex in the present embodiment is a complex in which the adjuvant is incorporated into the microparticles of a biodegradable polymer. In the complex, the adjuvant may be present inside the microparticles of the biodegradable polymer, or on the surface of the microparticles of the biodegradable polymer. At least one selected from the group consisting of chitosan, hexadecyltrimethylammonium, didodecyldimethylammonium, dimethyldioctadecylammonium, and salts thereof may be further incorporated into the first complex.

The average particle size of the complex comprising the adjuvant and the microparticles of the biodegradable polymer is preferably 5 nm to 800 nm, more preferably 10 nm to 500 nm, and even more preferably 50 nm to 200 nm. The average particle size can be measured by a dynamic light scattering method.

The second complex in the present embodiment is a complex in which the adjuvant is encapsulated in cyclodextrin. In the complex, one molecule of adjuvant may be encapsulated in one molecule of cyclodextrin, or in two or more molecules of cyclodextrin.

### (Adjuvant composition)

The adjuvant composition according to the present embodiment may further comprise an excipient in addition to the complex. Examples of the excipient include a sugar alcohol (e.g., mannitol), a disaccharide (e.g., trehalose, sucrose, lactulose, and maltose), a polysaccharide (e.g., crystalline cellulose), and an amino acid (e.g., glycine).

The excipient may be used alone or in combination of two or more.

The amount of excipient is preferably 10 to 99 mass%, more preferably 15 to 95 mass%, and even more preferably 20 to 90 mass% based on the adjuvant composition.

If the adjuvant composition according to the present embodiment comprises the first complex (i.e., a complex in which the adjuvant is incorporated into the microparticles of the biodegradable polymer), the adjuvant composition may further comprise at least one selected from the group consisting of alum, α-cyclodextrin, β-cyclodextrin, γ-cyclodextrin, and derivatives thereof. The derivatives of cyclodextrin are as described in the [Cyclodextrin] section above.

Examples of the form of the adjuvant composition include solid (e.g., freeze-dried products) and liquid (e.g., solutions and dispersions) forms.

Examples of the method for administering the adjuvant composition include injection administration, mucosal administration, and oral administration. Specific examples of the route of administration include subcutaneous, intradermal, intramuscular, inhalation, intranasal, sublingual, and oral.

Although not particularly limited, the complex in which the adjuvant is incorporated into the microparticles of the biodegradable polymer is preferably administered by injection (particularly, intramuscular injection).

Although not particularly limited, the complex in which the adjuvant is encapsulated in cyclodextrin is preferably mucosally administered (particularly, intranasally).

### <Vaccine composition>

One embodiment of the present invention relates to a vaccine composition comprising an adjuvant composition and a vaccine.

The details of the adjuvant composition in the present embodiment are as described in the <Adjuvant composition> section above, the contents of which are incorporated in the present embodiment by reference.

In the present specification, a "vaccine" means an agent that induces immunity (e.g., humoral and cellular immunity) to prevent the infection of a pathogen or onset of the disease.

Examples of the vaccine include a vaccine against adenovirus, AIDS virus, baculovirus, human cytomegalovirus (HCMV), hemorrhagic fever virus, hepatitis virus, herpes virus, immunodeficiency virus, T-cell leukemia virus, neonatal gastroenteritis virus, infectious hematopoietic necrosis virus, infectious pancreatic necrosis virus, influenza virus, Japanese encephalitis virus, leukemia virus, mumps virus, orthomyxovirus, pneumonia virus, poliovirus, polydnavirus, rotavirus, coronavirus, vaccinia virus, RS virus, Shigella species, Salmonella typhosa, tubercle bacillus, tetanus bacillus, diphtheria bacillus, meningococcus, Bordetella pertussis, Streptococcus pneumoniae, anthrax bacillus, bacillus botulinus, Clostridium difficile, Clostridium welchii, Enterococcus faecalis, Enterococcus faecium, hemophilus influenza, Helicobacter pylori bacteria, Mycobacterium leprae, gonococcus, meningococcus, Salmonella typhosa, Staphylococcus aureus, Treponema pallidum, cholera bacillus, falciparum malaria parasite, EB virus, hepatitis virus, T-cell lymphotropic virus, herpes virus, B virus, or papillomavirus.

Although not particularly limited, the vaccine is preferably a vaccine against influenza virus, coronavirus, RS virus, herpes virus, or papillomavirus.

Examples of the type of vaccine include a live vaccine, an inactivated vaccine, a chimeric vaccine, a recombinant protein vaccine, a peptide vaccine, a VLP vaccine, a nanoparticle vaccine, a toxoid vaccine, an mRNA vaccine, a replicon vaccine, a DNA vaccine, and a viral vector vaccine.

The vaccine may be used alone or in combination of two or more.

Examples of the form of the vaccine composition include solid (e.g., freeze-dried products) and liquid (e.g., solutions and dispersions) forms.

Examples of the method for administering the vaccine composition include injection administration, mucosal administration, and oral administration. Specific examples of the route of administration include subcutaneous, intradermal, intramuscular, inhalation, intranasal, sublingual, and oral.

Although not particularly limited, the vaccine composition containing the complex in which the adjuvant is incorporated into the microparticles of the biodegradable polymer is preferably administered by injection (particularly, intramuscular injection).

Although not particularly limited, the vaccine composition containing the complex in which the adjuvant is encapsulated in cyclodextrin is preferably mucosally administered (particularly, intranasally).

### <Method 1 for producing adjuvant composition>

One embodiment of the present invention relates to a method for producing an adjuvant composition, the method comprising incorporating an adjuvant into the microparticles of a biodegradable polymer to form a complex.

The details of the microparticles of biodegradable polymer, adjuvant, complex, and adjuvant composition in the present embodiment are as described in the <Adjuvant composition> section above, the contents of which are incorporated in the present embodiment by reference.

In forming the complex in the present embodiment, the formation of the microparticles of biodegradable polymer and the incorporation of the adjuvant may be carried out simultaneously, or the adjuvant may be incorporated into formed microparticles of biodegradable polymer.

The complex in the present embodiment can be produced, for example, by anemulsion solvent diffusion method. In an emulsion solvent diffusion method, the adjuvant and biodegradable polymer are dissolved in a good solvent, and the solution is added dropwise to a poor solvent under agitation. This allows the emulsification of the good solvent in the poor solvent, thereby forming emulsion drops of the good solvent. Furthermore, the interdiffusion between the good and the poor solvents allows the good solvent to continuously diffuse from within the emulsion to the poor solvent, which reduces the solubility of the adjuvant and biodegradable polymer in the emulsion drops, thereby forming a complex in which the adjuvant is incorporated into the microparticles of the biodegradable polymer.

The good solvent in an emulsion solvent diffusion method is a solvent in which the adjuvant and biodegradable polymer dissolve, and the poor solvent is a solvent in which the biodegradable polymer does not dissolve. The good solvent is preferably an organic solvent that is miscible in the poor solvent.

Examples of the poor solvent include water. When water is used as the poor solvent, it may be mixed with polyvinyl alcohol (PVA), lecithin, hydroxymethyl cellulose, or hydroxypropyl cellulose.

Examples of the good solvent include organic solvents that have low boiling points and are poorly soluble in water. Examples of such organic solvents include halogenated alkanes, acetone, methanol, ethanol, ethyl acetate, diethyl ether, cyclohexane, DMSO, benzene, and toluene.

The complex in the present embodiment can also be produced using a Shirasu porous glass membrane emulsification device, a microfluidic device, an inkjet device, or a forced thin film microreactor.

The adjuvant composition in the present embodiment and a vaccine can also be mixed to produce a vaccine composition. The details of the vaccine in the present embodiment are as described in the <Vaccine composition> section above, the contents of which are incorporated in the present embodiment by reference.

The adjuvant composition in the present embodiment can also be made into the form of a solid. Examples of the solidification method include a freeze-drying method, a spray-drying method, and an agitated vacuum drying method.

Examples of the freeze-drying method include a shelf-type freeze-drying method, a tube-type freeze-drying method, and a spray freeze-drying method. In the freeze-drying method, a sugar (e.g., trehalose, mannitol, and sucrose), an amino acid (e.g., glycine), a polysaccharide (e.g., crystalline cellulose), a salt (e.g., sodium chloride), or a buffer solution may be added, as necessary, to the liquid adjuvant composition, and the mixture is frozen and dried under reduced pressure. The adjuvant composition may be frozen and dried in a state in which the vaccine is mixed therein.

### <Method 2 for producing adjuvant composition>

One embodiment of the present invention relates to a method for producing an adjuvant composition, the method comprising encapsulating an adjuvant in a cyclodextrin to form a complex.

The details of the cyclodextrin, adjuvant, complex, and adjuvant composition in the present embodiment are as described in the <Adjuvant composition> section above, the contents of which are incorporated in the present embodiment by reference.

The complex in the present embodiment can be produced, for example, by an emulsification method, a saturated aqueous solution method, a kneading method, or a mixing and grinding method.

In the emulsification method, the adjuvant is added to a suspension of cyclodextrin and emulsified with a homogenizer to obtain a complex in which the adjuvant is encapsulated in cyclodextrin.

In the saturated aqueous solution method, a saturated aqueous solution of cyclodextrin and the adjuvant are mixed by stirring to precipitate an inclusion product. Water is then evaporated or the temperature is lowered to remove the precipitate, which is dried to obtain a complex in which the adjuvant is encapsulated in cyclodextrin.

In the kneading method, the adjuvant is added to a paste containing cyclodextrin and a small amount of water, and the mixture is kneaded well in a kneader to obtain a complex in which the adjuvant is encapsulated in cyclodextrin.

In the mixing and grinding method, cyclodextrin and the adjuvant are placed in a vibrating mill and ground to obtain a complex in which the adjuvant is encapsulated in cyclodextrin.

The adjuvant composition in the present embodiment and a vaccine can also be mixed to produce a vaccine composition. The details of the vaccine in the present embodiment are as described in the <Vaccine composition> section above, the contents of which are incorporated in the present embodiment by reference.

The adjuvant composition in the present embodiment can also be made into the form of a solid. The solidification method is as described in the <Method 1 for producing adjuvant composition > section above, the contents of which are incorporated in the present embodiment by reference.

### Examples

Hereinafter, the present invention will be described in more details using Examples and Comparative Examples, but the technical scope of the present invention is not limited thereto.

### <Materials>

(Antigen)
OVA: Ovalbumin(Sigma-Aldrich)
rHA: Recombinant influenza virus HA protein (A/Puerto Rico/8/1934) (Sino Biological Japan)

### (Adjuvant/Reagent)

Squalene (FUJIFILM Wako Pure Chemical Corporation)
α-Galactosylceramide (Tokyo Chemical Industry Co., Ltd.) Alum (Imject^{™} Alum, Thermo Fisher Scientific)
AddaVax^{TM} (InvivoGen)

### (Biodegradable polymer)

Poly(lactic-co-glycolic acid) (50:50) with a molecular weight of 10,000 (Sigma-Aldrich)
Poly(lactic-co-glycolic acid) (50:50) with a molecular weight of 50,000 (Sigma-Aldrich)
Poly(lactic acid) with a molecular weight of 20,000 (Taki Chemical Co., Ltd.)

### (Cyclodextrin)

Hydroxypropyl-β-cyclodextrin (FUJIFILM Wako Pure Chemical Corporation)
Methyl-β-cyclodextrin (FUJIFILM Wako Pure Chemical Corporation)
Dimethyl-β-cyclodextrin (Toronto Research Chemicals Inc.)

### (Excipient)

Mannitol (FUJIFILM Wako Pure Chemical Corporation)
Trehalose (FUJIFILM Wako Pure Chemical Corporation)
Sucrose (Nacalai Tesque Inc.)
Crystalline cellulose (Ceolus (registered trademark) PH-F20JP, Asahi Kasei Chemicals Corporation)

### (Others)

PVA (GOHSENOL EG40-P, The Nippon Synthetic Chemical Industry Co., Ltd.)
Dimethyldioctadecylammonium bromide (Tokyo Chemical Industry Co., Ltd.)

Details of each Example and Comparative Example are shown in Table 1-1 to Table 1-3.

**[Table 1-1]**

| | Adjuvant composition | | | | |
|---|---|---|---|---|---|
| | Adjuvant/Reagent | Biodegradable polymer | Cyclodextrin | Excipient | Remarks |
| Example 1 | Squalene | PLGA-1 | - | - | - |
| Example 2 | Squalene | PLGA-2 | - | - | - |
| Example 3 | αGC | PLGA-1 | - | - | - |
| Example 4 | Squalene | - | HP-β-CD | - | - |
| Example 5 | Squalene | - | M-β-CD | - | - |
| Example 6 | Squalene | PLGA-1 | - | Trehalose | Solidification |
| Example 7 | Squalene | PLGA-1 | - | Trehalose Mannitol | Solidification |
| Example 8 | Squalene | PLGA-1 | - | Sucrose Mannitol | Solidification |
| Example 9 | Squalene | PLGA-1 | - | Trehalose Crystalline cellulose | Solidification |
| Example 10 | Squalene | - | HP-β-CD | - | Solidification |
| Example 11 | Squalene | PLA | - | - | - |
| Comparative Example 1 | - | - | - | - | - |
| Comparative Example 2 | - | PLGA-1 | - | - | |
| Comparative Example 3 | Squalene | - | - | - | - |
| Comparative Example 4 | Alum | - | - | - | - |
| Comparative Example 5 | AddaVax | - | - | - | - |
| Comparative Example 6 | - | - | HP-β-CD | - | - |
| Comparative Example 7 | AddaVax | - | - | Trehalose | Solidification |
| Comparative Example 8 | AddaVax | - | - | Trehalose Mannitol | Solidification |
| Comparative Example 9 | AddaVax | - | - | Sucrose Mannitol | Solidification |

| | | | | | |
|---|---|---|---|---|---|
| αGC: α-Galactosylceramide PLGA-1: Poly(lactic-co-glycolic acid) (50:50) with a molecular weight of 10,000 PLGA-2: Poly(lactic-co-glycolic acid) (50:50) with a molecular weight of 50,000 PLA: Poly(lactic acid)with a molecular weight of 20,000 HP-β-CD:Hydroxypropyl-ρ-cyclodextrin M-β-CD:Methyl-β-cyclodextrin. | | | | | |

**[Table 1-2]**

| | Adjuvant composition | | | | |
|---|---|---|---|---|---|
| | Adjuvant/Reagent | Biodegradable polymer | Cyclodextrin | Excipient | Remarks |
| Example 12 | Squalene | PLGA-1 | - | - | - |
| Example 13 | Squalene | PLGA-1 | - | - | Contains alum |
| Example 14 | Squalene | PLGA-1 | - | - | Contains DM-β-CD |
| Example 15 | Squalene | PLGA-1 | - | - | Contains DDAB |
| Comparative Example 10 | - | - | - | - | - |
| Comparative Example 11 | Alum | - | - | - | - |

| | | | | | |
|---|---|---|---|---|---|
| PLGA-1: Poly(lactic-co-glycolic acid) (50:50) with a molecular weight of 10,000 DM-β-CD: Dimethyl-B-cyclodextrin DDAB: Dimethyldioctadecylammonium bromide | | | | | |

**[Table 1-3]**

| | Adjuvant composition | | | | |
|---|---|---|---|---|---|
| | Adjuvant/Reagent | Biodegradable polymer | Cyclodextrin | Excipient | Remarks |
| Example 16 | Squalene | PLGA-1 | - | - | - |
| Comparative Example 12 | - | - | - | - | - |
| Comparative Example 13 | AddaVax | - | - | - | - |

| | | | | | |
|---|---|---|---|---|---|
| PLGA-1: Poly(lactic-co-glycolic acid) (50:50) with a molecular weight of 10,000 | | | | | |

### <Production method>

### (Adjuvant composition comprising complex of squalene and biodegradable polymer)

For Example 1, Example 2, Examples 6 to 9, Example 11 and Comparative Example 2 in Table 1-1, and Example 16 in Table 1-3, 50 mL of a 0.05% PVA aqueous solution was collected in a 100-mL beaker, and while stirring this solution at a stirring speed of 1300 rpm, 10 mL of a mixture of 3 mg/mL squalene and 12 mg/mL PLGA solution or 12 mg/mL PLA solution dissolved in acetone/methanol (4/1) was injected at a rate of about 0.6 mg/min using a syringe pump (Micro Syringe Pump Economy, AS ONE Corporation). The prepared solution was filtered through a 1 µm filter, the filtrate was filtered through a 0.5 µm filter, and then the filtrate was further filtered through a 0.45 µm filter. 15 mL of the filtrate was collected in an Amicon Ultra-15 mL (Merck Millipore) and centrifuged at 4000 rpm for 330 min using a centrifuge (KUBOTA 3740, KUBOTA Manufacturing Corporation). The remaining liquid on the filter was recovered and used as the adjuvant solution composition. For Examples 6 to 9, the adjuvant solution composition was solidified by the solidification method described below. For Comparative Example 2, the same preparation as in Example 1 was performed, but without adding squalene.

For Example 12 in Table 1-2, 50 mL of a 0.05% PVA aqueous solution was collected in a 100-mL beaker, and while stirring this solution at a stirring speed of 1300 rpm, 10 mL of a mixture of 3 mg/mL squalene and 12 mg/mL PLGA dissolved in acetone was injected at a rate of about 0.6 mL/min using a syringe pump (Micro Syringe Pump Economy, AS ONE Corporation). The prepared solution was stirred overnight and centrifuged at 2000 x g for 10 min using a centrifuge (KUBOTA 3740/3700, KUBOTA Manufacturing Corporation), and the supernatant was recovered. The resulting supernatant solution was further centrifuged at 20000 x g for 20 min (high-speed centrifugation), the precipitate was dispersed in 1 mL ultrapure water, and then centrifuged at 1000 x g for 3 min. The resulting supernatant was used as the adjuvant solution composition.

For Examples 13 and 14 in Table 1-2, an alum suspension or a dimethyl-β-cyclodextrin solution was further added to the adjuvant solution composition prepared for Example 12, the mixture of which was used as the adjuvant solution composition.

For Example 15 in Table 1-2, 50 mL of a 0.05% PVA aqueous solution was collected in a 100-mL beaker, and while stirring this solution at a stirring speed of 1300 rpm, 11 mL of a mixture of 5.45 mg/mL squalene, 10.9 mg/mL PLGA and 2.73 mg/mL dimethyldioctadecylammonium bromide (DDAB) (Tokyo Chemical Industry) dissolved in acetone/methanol (10/1) was injected at a rate of about 0.6 mL/min. The prepared solution was stirred overnight and centrifuged at 2000 x g for 10 min using a centrifuge (KUBOTA 3740/3700, KUBOTA Manufacturing Corporation), and the supernatant was recovered. The resulting supernatant solution was further centrifuged at 20000 x g for 20 min (high-speed centrifugation), the precipitate was dispersed in 1 mL ultrapure water, and then centrifuged at 1000 x g for 3 min. The resulting supernatant was used as the adjuvant solution composition.

### (Adjuvant composition comprising complex of α-galactosylceramide and biodegradable polymer)

For Example 3 in Table 1-1, 50 mL of a 0.05% PVA aqueous solution was collected in a 100-mL beaker, and while stirring this solution at a stirring speed of 1300 rpm, 10 mL of a mixture of 0.45 mg/mL α-galactosylceramide and 12 mg/mL PLGA solution dissolved in acetone/methanol (4/1) was injected at a rate of about 0.6 mg/min using a syringe pump (Micro Syringe Pump Economy, AS ONE Corporation). The prepared solution was filtered through a 1 µm filter, the filtrate was filtered through a 0.5 µm filter, and then the filtrate was further filtered through a 0.45 µm filter. 15 mL of the filtrate was collected in an Amicon Ultra-15 mL (Merck Millipore) and centrifuged at 4000 rpm for 330 min using a centrifuge (KUBOTA 3740, KUBOTA Manufacturing Corporation). The remaining liquid on the filter was recovered and used as the adjuvant solution composition.

### (Adjuvant composition comprising complex of squalene and cyclodextrin)

For Examples 4, 5, and 10 in Table 1-1, 3.4 mL of physiological saline was added to 0.25 g of squalene and 1.5 g of cyclodextrin, and the mixture was sonicated using an ultrasonic cleaner (US-1, SND Co., Ltd.) until everything was liquefied before transferring all of the liquid into a test tube. To this test tube was added 1.6 mL of physiological saline, and the mixture was further sonicated for 90 minutes to obtain the adjuvant solution composition. For Example 10, the adjuvant solution composition was solidified by the solidification method described below.

### (Solidification of adjuvant solution composition)

For Examples 6 to 9 and Comparative Examples 7 to 9 in Table 1-1, 1 mL of diluted adjuvant solution composition or AddaVax^{TM} and a solution containing an excipient were added to a freeze-drying vial and placed in a shelf-type freeze-dryer (FreeZone Triad Freeze Dry System, Labconco Corp.) to obtain a freeze-dried product. The solutions containing the excipient were as follows: 4 mL of 10% trehalose solution for Example 6, 0.8 mL of 10% trehalose solution and 3.2 mL of 10% mannitol solution for Example 7, 0.8 mL of 10% sucrose solution and 3.2 mL of 10% mannitol solution for Example 8, 0.8 mL of 10% trehalose solution and 3.2 mL of 10% crystalline cellulose suspension for Example 9, 4 mL of 10% trehalose solution for Comparative Example 7, 0.8 mL of 10% trehalose solution and 3.2 mL of 10% mannitol solution for Comparative Example 8, and 0.8 mL of 10% sucrose solution and a 10% mannitol solution for Comparative Example 9. The freeze-drying conditions were as follows: after freezing at -30°C for 1 hour and -45°C for 1 hour, a primary drying of 60 hours was carried out at -35°C, and a secondary drying of 3 hours was further carried out at 30°C under a reduced pressure of 105 mTorr. The prepared freeze-dried product was ground and used as the adjuvant solid composition.

For Example 10 in Table 1-1, the prepared 5 mL adjuvant solution composition was solidified using a spray freeze-dryer (SFD-1100, Tokyo Rikakikai Co., Ltd). The spray freeze-drying conditions were as follows: flow rate of sample feeding into liquid nitrogen and spray pressure of 10 mL/min and 150 kPa, respectively, and a shelf temperature, reduced pressure, and time for drying of -100°C, 15 mTorr, and 24 hours or more, respectively.

### <Measurement of Average Particle Size>

The average particle size (Z-Average) of the adjuvant compositions containing a complex with a biodegradable polymer and of AddaVax^{TM}, an oil-in-water emulsion containing squalene, was measured using a particle size distribution analyzer based on dynamic light scattering (Zetasizer Nano ZS90, Spectris Co., Ltd.). The adjuvant solid composition was redissolved in ultrapure water before measuring the average particle size by the above method.

Table 2 shows the average particle size of the adjuvant solution compositions immediately after preparation. The average particle size of the adjuvant compositions containing a complex with a biodegradable polymer, that is, the average particle size before solidification without excipient for Examples 6 to 8 and the average particle size before adding alum or dimethyl-β-cyclodextrin for Examples 13 and 14, was about 200 nm.

**[Table 2]**

| | Average particle size (nm) |
|---|---|
| Example 1 | 219 |
| Example 2 | 187 |
| Example 3 | 201 |
| Example 6 | 238 |
| Examples 7, 8 | 229 |
| Example 11 | 181 |
| Examples 12-14 | 199 |
| Example 15 | 223 |
| Example 16 | 257 |
| Comparative Example 2 | 120 |
| Comparative Example 5 | 135 |
| Comparative Examples 7-9 | 143 |

Table 3 shows the average particle size of the adjuvant solid compositions of Examples 6 to 8 and Comparative Examples 7 to 9 after being redissolved by adding ultrapure water, and the difference from the average particle size before solidification with no excipient added. The results show that the effect on the average particle size before and after solidification was within ±10 nm for the compositions of the Examples, and that the effect on particle size was smaller than for the compositions of the Comparative Examples.

**[Table 3]**

| | Average particle size (nm) | Difference from before solidification (nm) |
|---|---|---|
| Example 6 | 240 | +2 |
| Example 7 | 220 | -9 |
| Example 8 | 219 | -10 |
| Comparative Example 7 | 290 | +147 |
| Comparative Example 8 | 228 | +85 |
| Comparative Example 9 | 221 | +78 |

### <Storage stability evaluation of adjuvant composition>

After adding 1 mL each of the adjuvant solution composition of Example 1 and AddaVax^{TM} (squalene-containing oil-in-water emulsion) of Comparative Example 5 to a clear glass vial and storing the glass vial with the lid on in a thermostatic chamber with the temperature set at 60°C for two weeks, the amount of squalene contained in the adjuvant solution composition and in AddaVax^{TM} was measured by HPLC and compared with the squalene content before storage. The HPLC measurement conditions were as follows: acetonitrile was used as the mobile phase, acetone, ethanol, or DMSO was added to the adjuvant solution composition and AddaVax^{TM}, and the mixture was sonicated, then diluted with acetone, methanol, or ethanol to an appropriate concentration, and filtered through a 0.45-µm syringe filter; the resulting filtrate was measured by HPLC (LC-2030C 3D plus, Shimadzu Corporation) to calculate the squalene content in the adjuvant solution composition and AddaVax^{TM}.

Table 4 shows the squalene content (%) after storage. The results show that the storage stability of squalene is superior in the adjuvant solution composition of Example 1 compared to AddaVax^{TM} of Comparative Example 5.

**[Table 4]**

| | Squalene content (%) |
|---|---|
| Example 1 | 98.7 |
| Comparative Example 5 | 87.3 |

### <Immunogenicity testing using OVA antigen in mice> (Subcutaneous administration and sampling)

To three 6-week-old female C57BL/6NCrl mice (Charles River Japan Co., Ltd.) per group, an administration solution of Example 1 containing the adjuvant solution composition of Example 1 listed in Table 1-1 (0.75 mg of squalene/3 mg of biodegradable polymer) and 50 µg of OVA antigen was subcutaneously administered on Day 0 and Day 7. As comparison groups, an administration solution of Comparative Example 1 containing 50 µg of OVA antigen without adjuvant composition as shown in Comparative Example 1 in Table 1-1; an administration solution of Comparative Example 2 containing a microparticle-containing solution (20 mg of biodegradable polymer) and 50 µg of OVA antigen, an administration solution of Comparative Example 3 containing 0.75 mg of squalene and 50 µg of OVA antigen , an administration solution of Comparative Example 4 containing 50 µg of alum and 50 µg of OVA antigen, and an administration solution of Comparative Example 5 containing 15 µL of AddaVax^{TM} (containing 0.75 mg of squalene) and 50 µg of OVA antigen were subcutaneously administered on Day 0 and Day 7. On Day 14, blood from the abdominal aorta and the spleen were collected under deep anesthesia to measure the anti-OVA-IgG antibodies and IFN-γ secretion by antigen restimulation, respectively.

### (Intranasal administration and sampling)

To three 6-week-old female C57BL/6NCrl mice (Charles River Japan Co., Ltd.) per group, an administration solution of Example 3 containing the adjuvant solution composition of Example 3 shown in Table 1-1 (2 µg of α-galactosylceramide/0.03 mg of biodegradable polymer) and 50 µg of OVA antigen, or an administration solution of Example 4 containing the adjuvant solution composition of Example 4 (0.75 mg of squalene/4.5 mg of cyclodextrin) and 50 µg of OVA antigen were intranasally administered on Day 0 and Day 7. As comparison groups, an administration solution of Comparative Example 1 containing 50 µg of OVA antigen without an adjuvant composition as shown in Comparative Example 1 in Table 1-1; an administration solution of Comparative Example 6 containing 20 mg of cyclodextrin and 50 µg of OVA antigen, an administration solution of Comparative Example 3 containing 0.75 mg of squalene and 50 µg of OVA antigen, and an administration solution of Comparative Example 5 containing 15 µL of AddaVax^{TM} (containing 0.75 mg of squalene) and 50 µg of OVA antigen were intranasally administered on Day 0 and Day 7. On Day 14, blood from the abdominal aorta and the spleen were collected under deep anesthesia to measure the anti-OVA-IgG antibodies and IFN-γ secretion by antigen restimulation, respectively. After collecting the spleen, a catheter was inserted through the center of the trachea to collect bronchoalveolar lavage fluid (BALF). The collected bronchoalveolar lavage fluid was used to measure anti-OVA-IgA antibodies.

### (Measurement of IFN-γ secretion level from splenocytes)

The collected spleen of each mouse was mashed using a syringe plunger in sterile PBS, and the cell suspension was passed through a 70 µm cell strainer (BD Biosciences) to collect the cells. After centrifugation, red blood cells were removed by adding red blood cell lysis solution (BD Pharma Lyse; BD Biosiences), and the cells washed with sterile PBS were used as mouse splenocytes for measurement. The splenocytes of each mouse were prepared at a concentration of 1 x 10⁵ cells/mL using assay medium: RPMI + 10% FBS + 100 IU/mL penicillin + 100 µg/mL streptomycin, of which 50 µL were seeded in the plate provided with the measurement kit (ELISpotPLUS for Mouse IFN-y; Mabtech). Next, 50 µL of 200 pg/mL OVA₂₅₇₋₂₆₄ peptide solution, which is a mouse MHC I epitope, was added as an antigenic stimulant, and the mixture was incubated at 37°C, 5% CO₂ for 16 hours. After incubation, each well was washed with sterile PBS, 100 µL of 1 µg/mL Detection Antibody (R4-6A-biotin; provided with the kit) was added to each well, and the mixture was allowed to stand for 2 hours at room temperature. Two hours later, each well was washed with sterile PBS, 100 µL of 1000-fold diluted Streptavidin-ALP (provided with the kit) was added to each well, and the mixture was allowed to stand for 1 hour at room temperature. One hour later, 100 µL of BCIP/NBT-plus solution filtered through the 0.45 µm filter provided with the kit was added to each well and the mixture was allowed to stand for 15 minutes at room temperature and under light shielding. After confirming the presence of spots, each well was washed with ultrapure water, the membrane was dried at room temperature and under light shielding, and the number of spots was counted using an ELISpot analyzer (ImmunoSpot S6 Analyzer, Cellular Technology Ltd.).

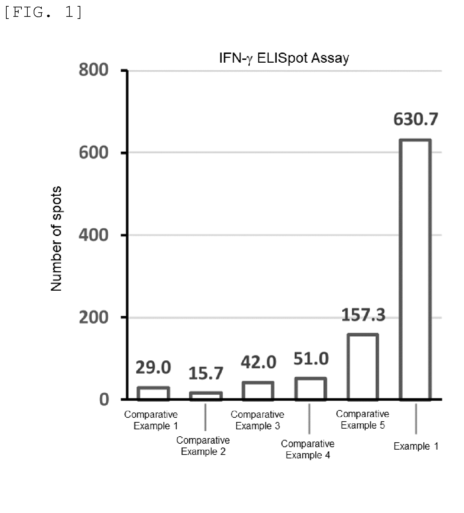

As shown in Figure 1, Example 1 was found to have a higher level of IFN-γ secretion (number of spots) compared to the Comparative Examples, i.e., a higher induction of cellular immunity.

### (Measurement of evaluation of IgG production level in blood)

100 µL of OVA solution prepared at 10 µg/mL with PBS was added to each well of a Nunc Immuno plate and allowed to stand for 16 hours at 4°C to immobilize the antigen. After immobilization, the plate was washed with PBS containing 0.05% polyoxyethylene sorbitan monolaurate (TWEEN 20), and 200 µL of blocking solution: PBS containing 2% w/v BSA was added to each well to block the antigen (room temperature, 1 hour). Next, 100 µL of serum sample diluted 8000-fold with a diluent: PBS containing 2% w/v BSA and 0.05% TWEEN 20 was added to each well and allowed to react (37°C 1 hour). After the reaction, the plate was washed, and 100 µL of secondary antibody solution (GOAT ANTI-MOUSE IGG HUMAN ADS-HRP; SOUTHERN BIOTECH) diluted 8000-fold with the diluent was added to each well (37°C 1 hour). After the reaction, the plate was washed, and 100 µL of TMB solution (Cell Signaling Technology, Inc.) was added to each well and allowed to react for 10 minutes at room temperature. Then, 100 µL of STOP solution (Cell Signaling Technology, Inc.) was added to each well to stop the reaction, and the absorbance of each well was measured at a wavelength of 450 nm using a plate reader (Multiskan Ascent, Thermo Fisher Scientific Inc.).

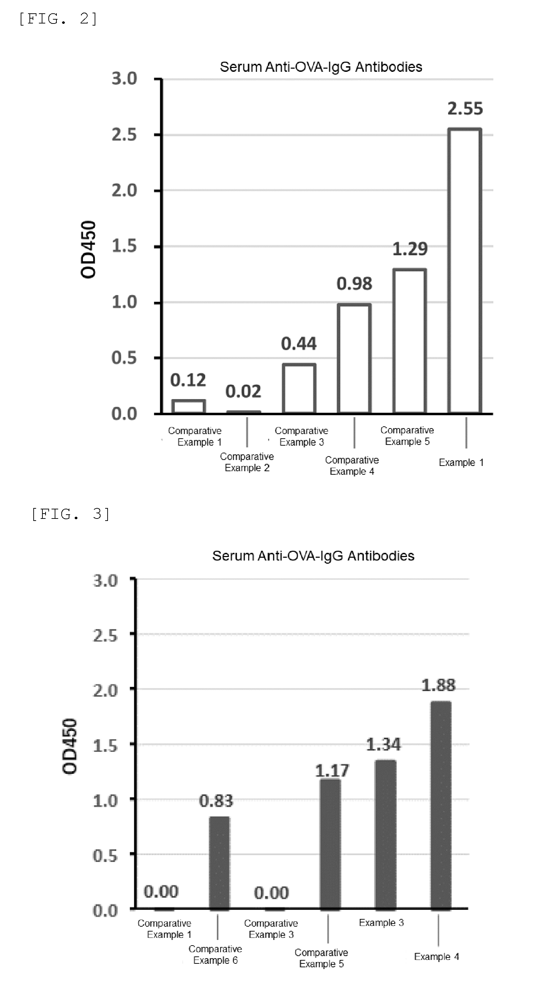

As shown in Figures 2 and 3, Example 1 delivered by subcutaneous administration and Examples 3 and 4 delivered by intranasal administration were found to have a higher serum anti-OVA-IgG antibody production compared to the Comparative Examples, i.e., a higher induction of humoral immunity in the blood.

### (Measurement of IgA production level in bronchoalveolar lavage fluid)

100 µL of OVA solution prepared at 10 µg/mL with PBS was added to each well of a Nunc Immuno plate and allowed to stand for 16 hours at 4°C to immobilize the antigen. After immobilization, the plate was washed with PBS containing 0.05% polyoxyethylene sorbitan monolaurate (TWEEN 20), and 200 µL of blocking solution: PBS containing 2% w/v BSA was added to each well to block the antigen (room temperature, 1 hour). Next, 100 µL of bronchoalveolar lavage fluid sample diluted 5-fold with a diluent: PBS containing 2% w/v BSA, 0.05% Tween 20 was added to each well and allowed to react (37°C 1 hour). After the reaction, the plate was washed, and 100 µL of secondary antibody solution (Goat Anti-Mouse IgA Human ads-HRP; Southern Biotech) diluted 8000-fold with the diluent was added to each well (37°C 1 hour). After the reaction, the plate was washed, and 100 µL of TMB solution (Cell Signaling Technology, Inc.) was added to each well and allowed to react for 10 minutes at room temperature. Then, 100 µL of STOP solution (Cell Signaling Technology, Inc.) was added to each well to stop the reaction, and the absorbance of each well was measured at a wavelength of 450 nm using a plate reader (Multiskan Ascent, Thermo Fisher Scientific Inc.).

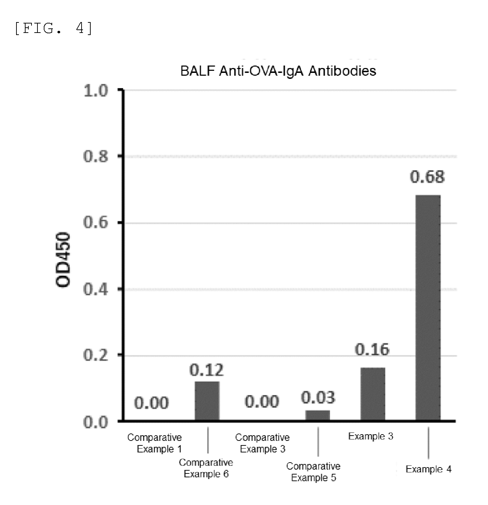

As shown in Figure 4, Examples 3 and 4 delivered by intranasal administration were found to have a higher anti-OVA-IgA antibody production in bronchoalveolar lavage fluid (BALF) compared to the Comparative Examples, i.e., in addition to the humoral immunity in the blood, the induction of humoral immunity in the mucosa was also higher.

### <Immunogenicity testing using rHA antigen in mice> (Subcutaneous administration and sampling)

Three 6 to 9-week-old female C57BL/6NCrl mice (Charles River Japan Co., Ltd.) per group were subcutaneously administered an administration solution of Example 12 containing the adjuvant solution composition of Example 12 in Table 1-2 (0.05 mg of squalene/0.20 mg of biodegradable polymer) and 3 µg of rHA antigen, an administration solution of Example 13 containing the adjuvant solution composition of Example 13 (0.05 mg of squalene/0.20 mg of biodegradable polymer/0.04 mg of alum) and 3 µg of rHA antigen, or an administration solution of Example 15 containing the adjuvant solution composition of Example 15 (0.05 mg of squalene/0.50 mg of biodegradable polymer/0.05 mg of dimethyldioctadecylammonium bromide) and 3 µg of rHA antigen on Day 0 and Day 7. As comparison groups, an administration solution of Comparative Example 10 containing 3 µg of rHA antigen without adjuvant composition as shown in Comparative Example 10 in Table 1-2, and an administration solution of Comparative Example 11 containing 0.04 mg of alum and 3 µg of rHA antigen were subcutaneously administered on Day 0 and Day 7. On Day 14, blood from the abdominal aorta was collected under deep anesthesia to measure the anti-rHA-IgG antibodies.

### (Measurement of evaluation of IgG production level in blood)

100 µL of rHA antigen solution prepared at 5 µg HA/mL with PBS was added to each well of a Nunc Immuno plate and allowed to stand for 16 hours at 4°C to immobilize the antigen. After immobilization, the plate was washed with PBS containing 0.05% polyoxyethylene sorbitan monolaurate (TWEEN 20), and 200 µL of blocking solution: PBS containing 2% w/v BSA was added to each well to block the antigen (room temperature, 1 hour). Next, 100 µL of serum sample diluted 8000-fold with a diluent: PBS containing 2% w/v BSA and 0.05% TWEEN 20 was added to each well and allowed to react (37°C 1 hour). After the reaction, the plate was washed, and 100 µL of secondary antibody solution (GOAT ANTI-MOUSE IGG HUMAN ADS-HRP; SOUTHERN BIOTECH) diluted 10000-fold with the diluent was added to each well (37°C 1 hour). After the reaction, the plate was washed, and 100 µL of TMB solution (Cell Signaling Technology, Inc.) was added to each well and allowed to react for 10 minutes at room temperature. Then, 100 µL of STOP solution (Cell Signaling Technology, Inc.) was added to each well to stop the reaction, and the absorbance of each well was measured at a wavelength of 450 nm using a plate reader (Infinite (registered trademark) 200Pro M Plex, TECAN Group Ltd.).

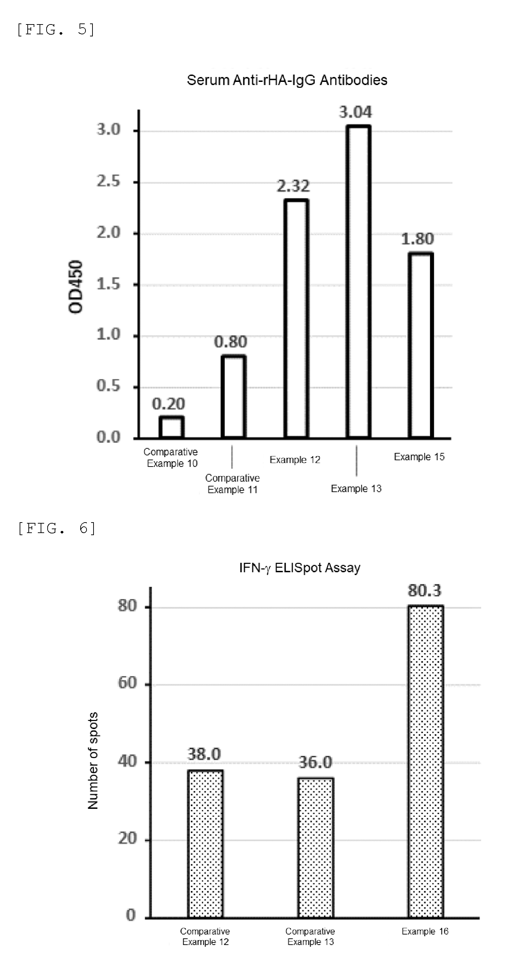

As shown in Figure 5, Examples 12, 13 and 15 were found to have a higher serum anti-rHA-IgG antibody production compared to the Comparative Examples, i.e., a higher induction of humoral immunity in the blood.

### <Immunogenicity testing using OVA antigen in monkeys>

### (Intramuscular administration and sampling)

Twelve 4- to 11-year-old male and female cynomolgus monkeys (SHIN NIPPON BIOMEDICAL LABORATORIES, LTD.) per group were intramuscularly administered an administration solution of Example 16 containing the adjuvant solution composition of Example 16 in Table 1-3 (4.875 mg of squalene/23.8 mg of biodegradable polymer) and 1 mg of OVA antigen on Day 0 and Day 21. As comparison groups, an administration solution of Comparative Example 12 containing 1 mg of OVA antigen without an adjuvant composition as shown in Comparative Example 12 in Table 1-3, and an administration solution of Comparative Example 13 containing 125 µL of AddaVax^{TM} (containing 4.875 mg of squalene) and 1 mg of OVA were intramuscularly administered on Day 0 and Day 21. On Day 42, blood was collected from the femoral vein, and serum and peripheral blood mononuclear cells were isolated to measure the anti-OVA-IgG antibodies and IFN-γ secretion by antigen restimulation, respectively.

### (Measurement of IFN-γ secretion level from peripheral blood mononuclear cells)

An equal volume of HBSS (Life Technologies Co) was added to 10 mL of the blood collected from each cynomolgus monkey. 15 mL of Ficoll-Paque Plus (GE Healthcare) was added to a SepMate tube (STEMCELL), to which each HBSS-added blood sample was gently added in layers and centrifuged for 30 minutes at 1000 G and room temperature. After centrifugation, 1 mL of the mononuclear cell layer was isolated with a micropipette, washed with sterile PBS, and used as peripheral blood mononuclear cells. The peripheral blood mononuclear cells of each cynomolgus monkey were prepared at a concentration of 1 x 10⁵ cells/mL using assay medium: RPMI + 10% FBS + 100 IU/mL penicillin + 100 µg/mL streptomycin, of which 50 µL were seeded in the plate provided with the measurement kit (ELISpotPLUS for Monkey IFN-y; Mabtech). Next, 50 µL of 200 µg/mL OVA solution was added as an antigenic stimulant, and the mixture was incubated at 37°C, 5% CO₂ for 16 hours. After incubation, each well was washed with sterile PBS, 100 µL of 1 µg/mL Detection Antibody (R4-6A-biotin; provided with the kit) was added to each well, and the mixture was allowed to stand for 2 hours at room temperature. Two hours later, each well was washed with sterile PBS, 100 µL of 1000-fold diluted Streptavidin-ALP (provided with the kit) was added to each well, and the mixture was allowed to stand for 1 hour at room temperature. One hour later, 100 µL of BCIP/NBT-plus solution filtered through the 0.45 µm filter provided with the kit was added to each well and the mixture was allowed to stand for 15 minutes at room temperature and under light shielding. After confirming the presence of spots, each well was washed with ultrapure water, the membrane was dried at room temperature and under light shielding, and the number of spots was counted using an ELISpot analyzer (ImmunoSpot S6 Analyzer, Cellular Technology Ltd.).

As shown in Figure 6, Example 16 was found to have a higher level of IFN-γ secretion (number of spots) compared to the Comparative Examples, i.e., a higher induction of cellular immunity.

### (Measurement of evaluation of IgG production level in blood)

100 µL of OVA solution prepared at 10 µg/mL with PBS was added to each well of a Nunc Immuno plate and allowed to stand for 16 hours at 4°C to immobilize the antigen. After immobilization, the plate was washed with PBS containing 0.05% polyoxyethylene sorbitan monolaurate (TWEEN 20), and 200 µL of blocking solution: PBS containing 2% w/v BSA was added to each well to block the antigen (room temperature, 1 hour). Next, 100 µL of serum sample diluted 8000-fold with a diluent: PBS containing 2% w/v BSA and 0.05% TWEEN 20 was added to each well and allowed to react (37°C 1 hour). After the reaction, the plate was washed, and 100 µL of secondary antibody solution (GOAT ANTI-Monkey IGG (FC specific) conjugated with HRP; Nordic-MU bio BV) diluted 10000-fold with the diluent was added to each well (37°C 1 hour). After the reaction, the plate was washed, and 100 µL of TMB solution (Cell Signaling Technology, Inc.) was added to each well and allowed to react for 10 minutes at room temperature. Then, 100 µL of STOP solution (Infinite (registered trademark) 200Pro M Plex, TECAN Group Ltd.) was added to each well to stop the reaction, and the absorbance of each well was measured at a wavelength of 450 nm using a plate reader (Multiskan Ascent, Thermo Fisher Scientific Inc.).

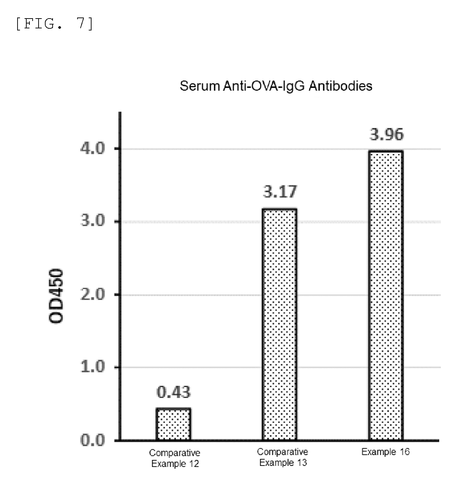

As shown in Figure 7, Example 16 was found to have a higher serum anti-OVA antibody production compared to the Comparative Examples, i.e., a higher induction of humoral immunity in the blood.

## Claims

1. An adjuvant composition comprising a complex comprising a terpenoid adjuvant and cyclodextrin,
wherein the terpenoid adjuvant is encapsulated in the cyclodextrin.

2. The adjuvant composition according to claim 1, wherein an amount of the terpenoid adjuvant is 0.1 to 40 mass% based on the complex.

3. The adjuvant composition according to claim 1 or 2, wherein the terpenoid adjuvant comprises at least one selected from the group consisting of retinoids and squalene.

4. The adjuvant composition according to any one of claims 1 to 3, wherein the cyclodextrin comprises at least one selected from the group consisting of α-cyclodextrin, β-cyclodextrin, γ-cyclodextrin, and derivatives thereof.

5. The adjuvant composition according to claim 4, wherein the derivative of the cyclodextrin is a methyl derivative, a dimethyl derivative, or a hydroxypropyl derivative.

6. The adjuvant composition according to any one of claims 1 to 5, further comprising at least one selected from the group consisting of crystalline cellulose, mannitol, trehalose, and sucrose.

7. The adjuvant composition according to any one of claims 1 to 6, which is in the form of a solid.

8. The adjuvant composition according to any one of claims 1 to 7 for use in mucosal administration.

9. A vaccine composition, comprising the adjuvant composition according to any one of claims 1 to 7, and a vaccine.

10. The vaccine composition according to claim 9, wherein the vaccine is a vaccine against influenza virus, coronavirus, RS virus, herpes virus, or papillomavirus.

11. A method for producing an adjuvant composition, the method comprising encapsulating a terpenoid adjuvant in a cyclodextrin to form a complex.

12. The method according to claim 11, wherein the complex is formed by an emulsification method, a saturated aqueous solution method, a kneading method, or a mixing and grinding method.
